# EUROPÄISCHE PATENTSCHRIFT

(11) **EP 2 475 317 B1**
(45) Veröffentlichungstag und Bekanntmachung des Hinweises auf die Patenterteilung: **23.01.2019**
(21) Anmeldenummer: 10757024.4
(22) Anmeldetag: 07.09.2010
(51) Int. Cl.: A61B 18/00, A61B 18/04, H01H 5/24

(54) **KARBONISIERUNGSVERHINDERUNGSVORRICHTUNG**
ANTI-CARBONISATION DEVICE
DISPOSITIF ANTI-CARBONISATION

(30) Priorität: 11.09.2009 DE 102009041168; 12.11.2009 DE 102009044512
(43) Veröffentlichungstag der Anmeldung: 18.07.2012
(73) Patentinhaber: Erbe Elektromedizin GmbH, 72072 Tübingen (DE)
(72) Erfinder: FISCHER, Klaus, 72202 Nagold (DE); NEUGEBAUER, Alexander, 72116 Mössingen (DE); ENDERLE, Markus, 72070 Tübingen (DE); ZENKER, Matthias, 72074 Tübingen (DE)
(74) Vertreter: Rüger Abel
(86) Internationale Anmeldenummer: PCT/EP2010/005485
(87) Internationale Veröffentlichungsnummer: WO 2011/029573

(56) Entgegenhaltungen:
- EP-A1- 1 829 492
- EP-A1- 2 160 081
- EP-A2- 0 740 926
- WO-A1-2006/119892
- WO-A1-2008/090004
- WO-A1-2009/060213
- WO-A1-2009/146432
- US-A1- 2004 116 918
- US-A1- 2005 118 350
- US-A1- 2007 029 500
- US-A1- 2009 039 790
- US-B1- 6 582 427

## Beschreibung

Die Erfindung betrifft eine Karbonisierungsverhinderungsvorrichtung zum Verhindern der Karbonisierung von Gewebe bei einer Plasma-Koagulation gemäß dem Oberbegriff des Patentanspruchs 1.

Die Hochfrequenzchirurgie, der auch die Argon-Plasma-Koagulation als Teilgebiet zuzuordnen ist, wird seit vielen Jahren sowohl in der Human- als auch in der Veterinärmedizin eingesetzt, um biologisches Gewebe zu koagulieren und/oder zu schneiden. Dabei wird mit Hilfe geeigneter elektrochirurgischer Instrumente hochfrequenter Strom durch das zu behandelnde Gewebe geleitet, so dass sich dieses aufgrund Eiweißkoagulation und Dehydration verändert. Es lassen sich somit aufgrund eines Koagulationsprozesses Gefäße verschließen und Blutungen stillen. Ein auf den Koagulationsprozess folgender Schneidprozess ermöglicht dann die vollständige Trennung bereits koagulierten Gewebes.

Die Argon-Plasma-Koagulation ermöglicht ein berührungsfreies Koagulieren von Gewebe und dient der effektiven Blutstillung und Gewebedevitaliserung. Bei dieser Koagulationsart wird inertes Arbeitsgas, z.B. Argon, über Gaszuführungseinrichtungen von einem Argon-Plasma-Koagulations-Instrument an das zu behandelnde Gewebe geleitet. Mit Hilfe des Arbeitsgases kann dann ein "Plasmastrahl" zwischen einer Elektrode an einem distalen Ende der Gaszuführungseinrichtung, beispielsweise einer Sonde, und dem Gewebe erzeugt werden. Der HF-Strom lässt sich dann an dem zu behandelnden Gewebe applizieren, ohne dass das elektrochirurgische Instrument mit dem Gewebe in Kontakt kommt. Ein Verkleben des Gewebes mit den Instrumenten wird somit vermieden.

Ein unerwünschter Nebeneffekt der Plasma-Koagulation, insbesondere der Argon-Plasma-Koagulation, ist die eintretende Karbonisierung des Gewebes, die bei nahezu allen elektrochirurgischen Anwendungen auftritt. Mit der Plasma-Koagulation ist eine chemisch unvollständige Verbrennung des biologischen Gewebes verbunden, die in erheblichem Maße zu einer Karbonisierung des Gewebes und zu einer Ruß- und Rauchgasentwicklung führt. Diese Nachteile treten bei der Argon-Plasma-Koagulation im Vergleich zu laserchirurgischen Verfahren zwar vermindert auf, dennoch kommt es zu einer Karbonisierung, mit der eine verstärkte Entzündung des Gewebes und vermehrt post-operative Probleme einhergehen. Insbesondere bei der Anwendung höherer Leistungen und längerer Applikationszeiten führt die Argon-Plasma-Chirurgie zu einer deutlichen Karbonisierung und damit zu einer gesundheitsschädlichen Emission von Ruß und Rauchgas sowie zu einer starken Geruchsbelästigung. Dadurch sind teure und komplizierte Absaugungseinrichtungen und Reinigungen des Operationssaals erforderlich. Ein weiterer Nachteil der Plasma-Koagulation ist, dass sich bei ihrer Anwendung relativ inhomogene Gewebeschädigungen abzeichnen, welche durch die konzentrierte Ausbildung von Strompfaden des Edelgasplasmas verursacht werden. Dabei treten Vertiefungen auf der Gewebeoberfläche auf, die bei höheren Leistungen und längeren Applikationszeiten stärker karbonisieren als die übrige Gewebeoberfläche.

Aus der USA 6,582,427 B1 ist ein elektrochirurgisches Instrument zur Plasmakogulation bekannt, bei dem ein Gas-Oxidationsmittel-Gemisch zur Erzeugung eines Gas-Oxidationsmittel-Plasmas benutzt wird. Dazu sind Zuführeinrichtungen für Oxidationsmittel und Gas sowie eine Elektrode zur Erzeugung eines Plasmas vorgesehen. Das Oxidationsmittel wird dem Gas vor der Plasmaerzeugung zugemischt. Alternativ kann das Oxidationsmittel auch durch einen zusätzlichen Einlass dem Argonplasma zugeführt werden. Damit wird durch Nutzung von Sauerstoff einer Karbonisierung entgegengewirkt.

Außerdem ist aus der EP 0 740 926 A2 eine elektrochirurgische Vorrichtung zur Erzeugung eines Lichtbogens bekannt. Die Vorrichtung weist eine rohrartige Kammer auf, durch die sich längs einer Hochfrequenz beaufschlagte Elektrode erstreckt. Außerdem werden der Kammer über zwei getrennte Rohre Kochsalzlösung und Luft zugeführt, so dass sich ein Aerosolstrahl ausbildet. Das entstehende Aerosol wird ionisiert und liefert somit einen gleichmäßigen besonders gut lokalisierbaren Lichtbogen.

Die genannten Rohre bilden eine Zerstäubungsvorrichtung.

Der Erfindung liegt daher die Aufgabe zugrunde, eine Karbonisierungsverhinderungsvorrichtung zu schaffen, die eine Karbonisierung von Gewebe während der Plasma-Koagulation vermeidet und die darüber hinaus für eine homogenere Gewebebehandlung sorgt.

Die Aufgabe der vorliegenden Erfindung wird durch eine Karbonisierungsverhinderungsvorrichtung mit den Merkmalen des Patentanspruchs 1 gelöst. Die Karbonisierungsverhinderungsvorrichtung dient zum Verhindern der Karbonisierung von Gewebe bei einer Plasma-Koagulation, wobei die Plasma-Koagulation mittels eines geeigneten chirurgischen Instruments durchgeführt wird. Das chirurgische Instrument weist eine Zuführung für ein Oxidationsmittel, eine Zuführung für ein Gas und eine Elektrode zur Erzeugung eines Plasmas auf. Weiterhin ist vorgesehen, dass durch die Karbonisierungsverhinderungsvorrichtung ein Gas-Oxidationsmittel-Gemisch zur Erzeugung eines Gas-Oxidationsmittel-Plasmas bereitgestellt wird. Auf diese Weise kann das Gas-Oxidationsmittel-Gemisch durch ein hochfrequentes elektrisches Wechselfeld zwischen der Elektrode und dem zu behandelnden Gewebe gezündet werden, sodass ein Plasma erzeugt wird, welches einerseits das eingeleitete Gas und andererseits das Oxidationsmittel aufweist. Durch das Zuführen eines Oxidationsmittels zu dem Gas wird eine Kühlung der Gewebeoberfläche bewirkt, wodurch die Karbonisierung des Gewebes in vorteilhafter Weise vermindert wird. Darüber hinaus oxidiert das Oxidationsmittel den bei der Plasma-Koagulation entstehenden Kohlenstoff und bewirkt dadurch auch eine verminderte Ruß- und Rauchgasentwicklung. Grundsätzlich sind daher als Oxidationsmittel alle Stoffe geeignet, die Kohlenstoff oxidieren können. Besonders vorteilhaft ist weiterhin, dass die Plasmaenergie durch den Eintrag des Oxidationsmittels gleichmäßiger über die gesamte Koagulationsfläche verteilt wird. Außerdem wird durch die hier vorgeschlagene Karbonisierungsverhinderungsvorrichtung eine Austrocknung (Desikkation) der Gewebeoberfläche wesentlich vermindert. Mit steigender Applikationszeit kommt es daher zu einem deutlich geringeren elektrischen Impedanzabfall des biologischen Gewebes, wodurch längere medizinisch-relevante Behandlungszeiten als bei der herkömmlichen Plasma-Koagulation möglich sind.

Besonders bevorzugt wird eine Ausführungsform der Karbonisierungsverhinderungsvorrichtung, bei der das Oxidationsmittel flüssig oder gasförmig ist. Vorzugsweise wird als Oxidationsmittel Wasser und als Gas ein Inertgas, insbesondere Argon eingesetzt. Das Oxidationsmittel kann auch in Form eines Aerosols vorliegen, sodass es also in feine Oxidationsmitteltröpfchen zu einem Oxidationsmittelnebel zerstäubt ist. Durch den Oxidationsmittelnebel werden die spezifische Oberfläche und damit die Wärmeaustauschfläche zwischen dem Oxidationsmittel und dem Trägergas um mehr als das Hundertfache vergrößert, sodass der Verdampfungspunkt der flüssigen Oxidationsmitteltröpfchen erheblich herabgesetzt ist, der Oxidationsmittelnebel also schneller verdampft. Dadurch liegt ein erheblicher Anteil des Oxidationsmittels auch als Oxidationsmitteldampf vor. Auf diese Weise kann ein Teil des Oxidationsmittels, nämlich der gasförmig vorliegende Teil zu einem Oxidationsmitteldampfplasma ionisiert werden. Hierbei bildet sich ein reaktives Plasma, welches im Fall von Wasser als Oxidationsmittel Spezies wie H₂0⁺, H, OH und O Radikale enthält. Durch die Erhöhung der spezifischen Oberfläche kann die Gewebeoberfläche im Übrigen deutlich gekühlt werden, wodurch eine Karbonisierung reduziert wird. Denkbar ist es auch, das Oxidationsmittel vor der Bereitstellung des Gas-Oxidationsmittel-Gemisches mittels eines Verdampfers in seinen gasförmigen Zustand zu überführen. Weiterhin können dem Oxidationsmittel Nanopartikel mit bestimmten Eigenschaften beigemischt werden, die beispielsweise eine therapeutische Wirkung verstärken oder beschleunigen oder auch Nebenwirkungen verringern können. Denkbar ist es beispielsweise, Nanopartikel beizumischen, die den Wundheilungsprozess positiv beeinflussen.

Weiterhin bevorzugt wird eine Ausführungsform der Karbonisierungsverhinderungsvorrichtung, bei der das chirurgische Instrument zur Erzeugung des Aerosols einen Verdampfer aufweist. Weiterhin kann vorgesehen sein, dass statt eines Verdampfers eine Ultraschallerzeugungseinrichtung zur Erzeugung des Aerosols vorgesehen ist. Alternativ dazu kann jedoch auch eine Prallfläche vorgesehen sein, auf die das Oxidationsmittel prallt, so dass es beim Abprallen von der Fläche zerstäubt wird. Auf diese Weise kann das Gas-Oxidationsmittel-Gemisch besonders einfach durch die Karbonisierungsverhinderungsvorrichtung bereitgestellt werden.

Auch wird eine Ausführungsform einer Karbonisierungsverhinderungsvorrichtung bevorzugt, die sich dadurch auszeichnet, dass wenigstens eine Zweistoffzerstäubereinrichtung bzw. Zweistoffdüse vorgesehen ist. Diese kann innenmischend oder außenmischend ausgebildet sein. Durch die Zweistoffzerstäubungseinrichtung ist es auf einfache Art und Weise möglich, ein Gas-Oxidationsmittel-Gemisch zur Erzeugung eines Gas-Oxidationsmittel-Plasmas bereitzustellen.

Schließlich wird eine Ausführungsform einer Karbonisierungsverhinderungsvorrichtung bevorzugt, die sich dadurch auszeichnet, dass das chirurgische Instrument einen Schlauch aufweist, der im Bereich der Elektrode wenigstens eine Öffnung zur Verhinderung einer Gasembolie aufweist. Zumindest wird dadurch die Wahrscheinlichkeit für eine Gasembolie oder eine Emphysembildung bei Kontakt mit dem Gewebe wesentlich reduziert.

Erfindungsgemäß ist vorgesehen, dass eine selbstansaugende Zweistoffzerstäubereinrichtung vorgesehen ist, die vorzugsweise durch die Anordnung des Gaszufuhrkanals und des Oxidationsmittelzufuhrkanals geschaffen wird und die eine zusätzliche Pumpe für die Zufuhr des Oxidationsmittels überflüssig macht.

Die Aufgabe der vorliegenden Erfindung wird auch durch ein Verfahren zur Verhinderung der Karbonisierung von Gewebe bei der Plasma-Koagulation mit den Merkmalen des Anspruchs 13 gelöst. Das chirurgische Instrument weist vorzugsweise eine Zuführung für ein Oxidationsmittel, eine Zuführung für ein Gas und eine Elektrode zur Erzeugung eines Plasmas auf. Das Verfahren zeichnet sich durch folgenden Schritt aus: Bereitstellen eines Gas-Oxidationsmittel-Gemisches zur Erzeugung eines Gas-Oxidationsmittel-Plasmas. Durch das hier angesprochene vorteilhafte Verfahren kann eine Karbonisierung des Gewebes wesentlich vermindert werden, da der entstehende Kohlenstoff durch das Oxidationsmittel oxidiert wird. Darüber hinaus findet gleichzeitig eine Kühlung der Oberfläche des Gewebes durch das Oxidationsmittel statt. Besonders bevorzugt wird dabei ein Oxidationsmittel, welches flüssig oder gasförmig ist. Es kann jedoch auch vorgesehen sein, dass das Oxidationsmittel als Aerosol vorliegt. In diesem Fall weist das chirurgische Instrument vorzugsweise eine entsprechende Vorrichtung zur Erzeugung des Aerosols auf. Das Oxidationsmittel muss geeignet sein um Kohlenstoff zu oxidieren, was beispielsweise auf Wasser zutrifft. Als Gas wird vorzugsweise ein Inertgas, besonders bevorzugt Argon, eingesetzt.

Die Aufgabe der Erfindung wird schließlich durch die Verwendung einer Karbonisierungsverhinderungsvorrichtung gemäß den Merkmalen des Anspruchs 19 gelöst.

Durch die Verwendung einer Karbonisierungsverhinderungsvorrichtung, die ein Gas-Oxidationsmittel-Gemisch zur Erzeugung eines Gas-Oxidationsmittel-Plasmas bereitstellt, wird auf vorteilhafte Weise eine Karbonisierung des Gewebes vermindert.

Die Erfindung wird im Folgenden anhand der Zeichnung näher erläutert. Es zeigen:
Figur 1 eine schematische Schnittdarstellung einer ersten Ausführungsform einer Karbonisierungsverhinderungsvorrichtung;
Figur 2 eine schematische Schnittdarstellung einer zweiten Ausführungsform einer Karbonisierungsverhinderungsvorrichtung;
Figur 3 eine schematische Schnittdarstellung einer dritten Ausführungsform einer Karbonisierungsverhinderungsvorrichtung;
Figur 4 eine perspektivische Darstellung einer vierten Ausführungsform einer Karbonisierungsverhinderungsvorrichtung;
Figur 5 eine perspektivische Darstellung einer fünften Ausführungsform einer Karbonisierungsverhinderungsvorrichtung;
Figur 6 eine schematische Darstellung eines chirurgischen Instruments mit einer Elektrode und einer Zweistoffdüse;
Figur 7 eine Draufsicht auf einen Austrittsbereich eines chirurgischen Instruments, und
Figur 8 eine schematische Schnittdarstellung einer Ausführungsform eines chirurgischen Instruments mit Venturi-Düse.

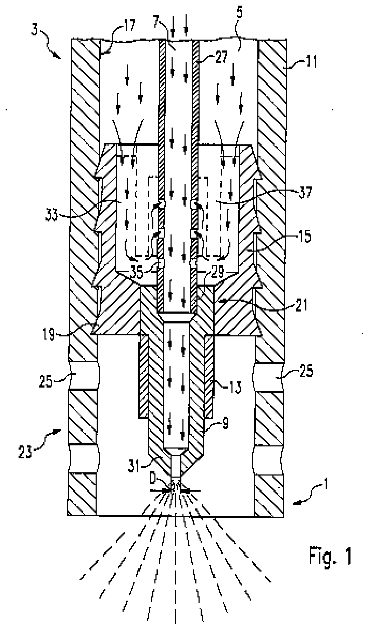

Figur 1 zeigt eine schematische Schnittdarstellung einer ersten Ausführungsform einer Karbonisierungsverhinderungsvorrichtung 1 gemäß der Erfindung. Sie dient dazu, eine Karbonisierung von Gewebe bei einer Plasma-Koagulation zu verringern, vorzugsweise ganz zu verhindern, wobei die Plasma-Koagulation mittels eines geeigneten chirurgischen Instruments 3 durchgeführt wird.

Das chirurgische Instrument 3 weist eine Zuführung 5 für ein Gas auf, im Folgenden Gaszufuhrkanal 5 genannt, sowie eine Zuführung 7 für ein Oxidationsmittel, im Folgenden Oxidationsmittelzufuhrkanal 7 genannt. Weiterhin ist eine Elektrode bzw. eine Elektrodenspitze 9 vorgesehen, die an eine hier nicht gezeigte Hochfrequenzspannungsquelle angeschlossen ist, die der Elektrodenspitze 9 einen hochfrequenten Strom zuführt. Die Elektrodenspitze 9 ist im Übrigen hohl ausgebildet und bildet somit quasi eine Verlängerung des Oxidationsmittelzufuhrkanals 7.

Der Gaszufuhrkanal 5, der Oxidationsmittelzufuhrkanal 7 und die Elektrode 9 sind gemäß Figur 1 beispielhaft innerhalb eines Schlauchs 11 angeordnet, der vorzugsweise aus PTFE besteht und der mit einem hier nicht gezeigten HF-Chirurgiegerät verbunden ist.

Figur 1 macht noch deutlich, dass ein Isolationsschutz 13 vorgesehen ist, der die Elektrodenspitze 9 zumindest bereichsweise koaxial umschließt. Ferner ist im Inneren des Schlauchs 11 eine Fixierhülse 15 vorgesehen, die an der Innenwandung 17 des Schlauchs 11 mittels geeigneter Rastvorsprünge 19 befestigt ist und die in einem distalen Bereich 21 die Elektrodenspitze 9 umschließt.

Das die Elektrodenspitze 9 umgebende distale Ende 23 des Schlauchs 11 weist im Übrigen laterale Öffnungen 25 auf, durch die das Gas-Oxidationsmittel-Gemisch entweichen kann, wodurch eine Gasembolie und eine Emphysembildung vermieden werden sollen, wenn das distale Ende 23 des Schlauchs 11 in Kontakt mit dem Gewebe kommt.

Der Oxidationsmittelzufuhrkanal 7 ist in einem Rohr 27 ausgebildet, welches vorzugsweise aus rostfreiem Stahl, insbesondere aus V2A-Stahl besteht. Das Rohr 27 ist an einem nicht gezeigten proximalen Ende mit einer HF-Spannungsquelle verbunden und dient somit gleichzeitig als elektrischer Leiter, der die Elektrodenspitze 9 mit einem hochfrequenten Strom versorgt. Hierzu ist das distale Ende 29 des Rohrs 27 mit der Elektrodenspitze 9 verbunden.

Weiterhin ist das Rohr 27 mit einer hier nicht gezeigten Oxidationsmittelquelle verbunden, so dass ein Oxidationsmittel durch das Rohr 27 und durch die Elektrodenspitze 9 zu einem distalen Ende 31 der Elektrodenspitze 9 gelangen kann.

Figur 1 macht noch deutlich, dass zwischen der Fixierhülse 15 und dem Rohr 27 ein Ringraum 33 vorgesehen ist, in den das Gas aus dem Gaszufuhrkanal 5 geleitet wird. Weiterhin weist das Rohr 27 im Bereich des Ringraums 33 wenigstens eine, hier mehrere Öffnungen 35 auf, durch die das Gas aus dem Ringraum 33 in den Oxidationsmittelzufuhrkanal 7 strömen kann. In dem Ringraum 33 kann im Übrigen ein Diffusor 37 angeordnet sein, der in Figur 1 gestrichelt dargestellt ist.

Der Gaszufuhrkanal 5 und der Oxidationsmittelzufuhrkanal 7 des chirurgischen Instruments 3 bilden somit zusammen eine Zweistoffdüse, die innenmischend ausgebildet ist, sodass also das Gas und das Oxidationsmittel getrennt einer Mischkammer zugeführt werden, wobei die Mischkammer bei der vorliegenden Ausführungsform durch den Oxidationsmittelzufuhrkanal 7 gebildet wird. Erst nach dem Mischen wird das Gas-Oxidationsmittel-Gemisch durch eine Düse nach außen geleitet, wobei die Düse vorliegend durch das distale Ende 31 der Elektrodenspitze 9 ausgebildet ist. Das distale Ende 31 kann hierzu einen bestimmten Innendurchmesser D und eine geeignete Form aufweisen, um eine gewünschte Strahlbreite des ausgestoßenen Gas-Oxidationsmittel-Gemisches zu erzeugen.

Auf diese Weise wird das Gas-Oxidationsmittel-Gemisch beim Austreten aus dem Oxidationsmittelzufuhrkanal 7 zerstäubt, sodass das Oxidationsmittel bzw. das Gas-Oxidationsmittel-Gemisch als Aerosol vorliegt. Um eine Plasma-Koagulation durchzuführen, wird die Elektrodenspitze 9 in die Nähe des zu behandelnden Gewebes gebracht und das austretende zerstäubte Gas-Oxidationsmittel-Gemisch durch die Elektrodenspitze 9 bzw. den dort anliegenden hochfrequenten Strom gezündet, sodass zwischen der Gewebeoberfläche und der Elektrodenspitze 9 ein leitfähiges Gas-Oxidationsmittel-Plasma entsteht, durch das der hochfrequente Strom von der Elektrodenspitze 9 zu dem Gewebe fließen kann, um dort eine Koagulation des Gewebes zu bewirken.

Die oben beschriebene Karbonisierungsverhinderungsvorrichtung 1 wird besonders bevorzugt bei der Argon-Plasma-Koagulation eingesetzt. Als Gas wird also vorzugsweise Argon verwendet, welches durch den Gaszufuhrkanal 5, den Ringraum 33 und die Öffnungen 35 dem Oxidationsmittel zugeführt wird. Als Oxidationsmittel kann jeder Stoff dienen, der geeignet ist Kohlenstoff zu oxidieren. Vorzugsweise wird jedoch Wasser als Oxidationsmittel eingesetzt, welches den bei der Plasma-Koagulation entstehenden Kohlenstoff gemäß der folgenden Formel oxidiert:

131.38 kJ/mol + C + H20(g) → CO + H2 (1)

CO + H2Q(g) → CQ2 + H2 + 41.19 kJ/mol (2)

90.19 kJ/mol + C + 2 H20(g) → C02 + 2 H2 (3)

Das Oxidationsmittel kann flüssig oder gasförmig in den Oxidationsmittelzufuhrkanal 7 geleitet werden. Falls das Oxidationsmittel in flüssiger Form in den Oxidationsmittelzufuhrkanal 7 geleitet wird, ist vorzugsweise vorgesehen, dass das Oxidationsmittel durch geeignete Mittel in ein Aerosol überführt wird. Auch kann der Eintrag des Oxidationsmittels in der jeweiligen gasförmigen Substanz erfolgen, wobei das gasförmige Oxidationsmittel vorher beispielsweise mittels eines Verdampfers erzeugt wurde.

In der Ausführungsform gemäß Figur 1 ist beispielsweise vorgesehen, dass das Oxidationsmittel in flüssiger Form durch den Oxidationsmittelzufuhrkanal 7 geleitet wird und mit dem Gas aus dem Gaszufuhrkanal 5 versetzt wird. Das Gas-Oxidationsmittel-Gemisch wird anschließend der Düse in dem distalen Ende 31 der Elektrodenspitze 9 zugeführt. Auf diese Weise wird das Gas-Oxidationsmittel-Gemisch zerstäubt, sodass es nach dem Austreten aus der Elektrode als Aerosol vorliegt, wo es durch den HF-Strom zu einem Plasma gezündet wird. Es liegt somit ein Gas-Oxidationsmittel-Plasma vor.

Untersuchungen haben in diesem Zusammenhang im Übrigen gezeigt, dass die Anwesenheit eines feinen Oxidationsmittelnebels, insbesondere eines Wassernebels zu einem besseren Zünden des Gas-Oxidationsmittel-Gemisches führen.

Das Oxidationsmittel wird zur Erzeugung eines Gas-Oxidationsmittel-Plasmas, wie oben beschrieben, vorzugsweise in feine Tröpfchen zerstäubt, wodurch die spezifische Oberfläche des Oxidationsmittels wesentlich erhöht wird. Gleichzeitig resultiert hieraus eine wesentliche Verminderung des Verdampfungspunkts des Oxidationsmittels, sodass ein Teil des Oxidationsmittels in dem Plasma schneller in seinen gasförmigen Zustand übergeht. Der gasförmige Teil kann dann durch das vorhandene Gas-Plasma zu einem Oxidationsmitteldampfplasma ionisiert werden und trägt so zu der Leitung des Stroms von der Elektrode zu der Gewebeoberfläche bei. Das Zünden des gasförmigen Oxidationsmittels wird dabei durch das bereits vorhandene Plasma, insbesondere durch ein Argon-Plasma unterstützt. Durch das Zerstäuben des Oxidationsmittels bzw. des Gas-Oxidationsmittel-Gemisches dient somit zumindest ein Teil des Oxidationsmittels als Plasmamedium.

Das Gas-Oxidationsmittel-Plasma weist somit das ionisierte Gas, zerstäubtes Oxidationsmittel, d.h. kleine Oxidationsmitteltröpfchen, sowie ionisiertes Oxidationsmittel auf. Falls als Trägergas Argon und als Oxidationsmittel Wasser eingesetzt wird, resultiert aus dem oben Beschriebenen ein Plasma, welches positiv geladene Argonkationen, positiv geladene Wasserdampfradikalkationen sowie radikalische Spezies wie H, OH und O aufweist.

Insgesamt ergeben sich durch das Bereitstellen eines Gas-Oxidationsmittel-Gemisches folgende Vorteile:
Das zerstäubte Gas-Oxidationsmittel-Gemisch weist eine erhöhte spezifische Oberfläche auf, sodass die Oxidationsmitteltröpfchen die Gewebeoberfläche während der Plasma-Koagulation kühlen. Hierdurch wird eine Verminderung der Karbonisierung des Gewebes herbeigeführt.

Weiterhin führt die Erhöhung der spezifischen Oberfläche zu einer Herabsetzung des Verdampfungspunktes des Oxidationsmittels, so dass zumindest ein Teil des Oxidationsmittels verdampft, also gasförmig wird. Der gasförmige Teil kann dann durch das elektrische Wechselfeld ionisiert werden und bildet ein leitfähiges Oxidationsmitteldampfplasma. Der hochfrequente Wechselstrom, der durch das leitfähige Plasma geleitet wird ist für die Entstehung der Joul'schen Wärmeenergie im Zuge der zu verrichtenden Eintrittsarbeit in das biologische Gewebe verantwortlich, was wiederum als Nebeneffekt zu Erhitzung des biologischen Gewebes mit der entsprechend erwünschten therapeutischen Wirkung führt. Das Oxidationsmittel trägt daher in seinem ionisierten Zustand zu der therapeutischen Wirkung bei.

Schließlich oxidiert das Oxidationsmittel, insbesondere die flüssigen Oxidationsmitteltröpfchen in dem Plasma, den während der Karbonisierung entstehenden Kohlenstoff, wodurch die Karbonisierung und die Emission von Ruß und Rauch vermindert werden.

Insgesamt ist somit festzuhalten, dass das Gas-Oxidationsmittel-Gemisch vorzugsweise als Aerosol vorliegt, wobei das Gas also mit dem zerstäubten Oxidationsmittel vermischt ist. Auf diese Weise dient das Oxidationsmittel gleichzeitig als Kühlmittel für die Gewebeoberfläche, als Oxidationsmittel für Kohlenstoff und als Plasmamedium für die Leitung des hochfrequenten Stroms von der Elektrodenspitze 9 zu dem Gewebe.

Darüber hinaus wird durch die vorliegende Karbonisierungsverhinderungsvorrichtung 1 eine gleichmäßigere Verteilung der Plasmaenergie über die Koagulationsfläche erreicht, sodass konzentrierte Strompfade vermieden werden.

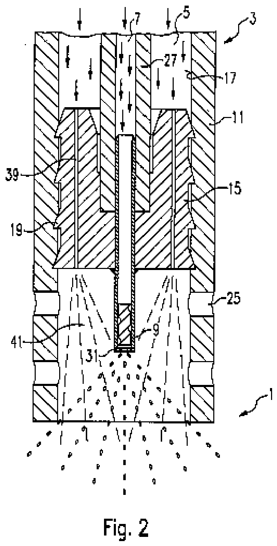

Figur 2 zeigt eine schematische Schnittdarstellung einer zweiten Ausführungsform einer Karbonisierungsverhinderungsvorrichtung 1 gemäß der Erfindung. Gleiche Teile sind mit gleichen Bezugszeichen versehen, sodass insofern auf die Beschreibung zu Figur 1 verwiesen wird, um Wiederholungen zu vermeiden.

Das chirurgische Instrument 3 gemäß Figur 2 weist wiederum einen Gaszufuhrkanal 5 und einen Oxidationsmittelzufuhrkanal 7 auf, die in dem Schlauch 11 angeordnet sind. Entsprechend der Ausführungsform gemäß Figur 1 ist darüber hinaus eine Fixierhülse 15 vorgesehen, die koaxial in dem Schlauch 11 angeordnet ist und mit geeigneten Rastvorsprüngen 19 an der Innenwandung 17 des Schlauchs 11 befestigt ist. In dem Schlauch 11 sind wiederum seitliche Öffnungen 25 vorgesehen, um eine Gasembolie zu vermeiden.

Weiterhin ist die Elektrodenspitze 9 im Wesentlichen zentrisch in der Fixierhülse 15 gelagert und mit dem Rohr 27 verbunden, welches als Oxidationsmittelzufuhrkanal 7 dient. Die Elektrodenspitze 9 ist wiederum hohl ausgebildet und dient quasi als Verlängerung des Oxidationsmittelzufuhrkanals 7. Das distale Ende der Elektrodenspitze 9 ist auch bei der Ausführungsform gemäß Figur 2 als Düse mit einem geeigneten Durchmesser und einer geeigneten Form ausgebildet, so dass das in dem Oxidationsmittelzufuhrkanal 7 geführte Oxidationsmittel beim Austreten aus dem Kanal zerstäubt wird.

Entgegen der in Figur 1 gezeigten Ausführungsform ist in Figur 2 vorgesehen, dass die Zweistoffdüse, die durch den Gaszufuhrkanal 5 und den Oxidationsmittelzufuhrkanal 7 gebildet wird, außenmischend ausgebildet ist. Das Gas und das Oxidationsmittel werden also nicht einer gemeinsamen Mischkammer zugeführt und dann zerstäubt, sondern das Gas und das Oxidationsmittel werden in zwei getrennten Kanälen nach außen geleitet und bilden erst nach dem Austreten aus ihren jeweiligen Zufuhrkanälen 5 und 7 ein Gas-Oxidationsmittel-Gemisch. Hierzu ist in der Fixierhülse 15 wenigstens eine axiale Durchgangsbohrung 39 vorgesehen, welche die Gaszufuhrleitung 5 mit einem Austrittsbereich 41 verbindet, in den die Elektrodenspitze 9 aus der Fixierhülse 15 hineinragt. In Figur 2 sind in der Schnittdarstellung zwei Durchgangsbohrungen 39 erkennbar. Denkbar ist es jedoch auch, einen Ringraum vorzusehen bzw. die Fixierhülse 15 zweiteilig auszubilden, sodass das Gas durch den Ringraum in den Austrittsbereich 41 gelangt.

Somit wird das Gas-Oxidationsmittel-Gemisch erst im Austrittsbereich 41 und nicht schon im Oxidationsmittelzufuhrkanal 7 gemäß Figur 1 bereitgestellt. Es kann auch vorgesehen sein, dass das Gas und das Oxidationsmittel im Austrittsbereich 41 am distalen Ende 31 der Elektrodenspitze 9 so zusammenwirken, dass durch das Aufeinandertreffen des Gases und des Oxidationsmittels ein Zerstäuben des Oxidationsmittels herbeigeführt wird. Auf eine Zerstäuberdüse kann dann verzichtet werden.

Auch bei der Ausführungsform gemäß Figur 2 kann das Oxidationsmittel flüssig oder gasförmig sein. Beispielsweise ist es denkbar das Oxidationsmittel schon gasförmig durch den Oxidationsmittelzufuhrkanal 7 zu leiten. Vorzugsweise liegt das Oxidationsmittel im Austrittsbereich 41 jedoch als Aerosol vor. Um ein Aerosol zu erzeugen, weist das chirurgische Instrument 3 vorzugsweise einen Verdampfer oder Erhitzer auf. Weiterhin kann ein Aerosol durch eine Ultraschallerzeugungseinrichtung erzeugt werden. Denkbar ist jedoch auch der Einsatz einer Prallfläche, an der das Oxidationsmittel abprallt und zerstäubt wird.

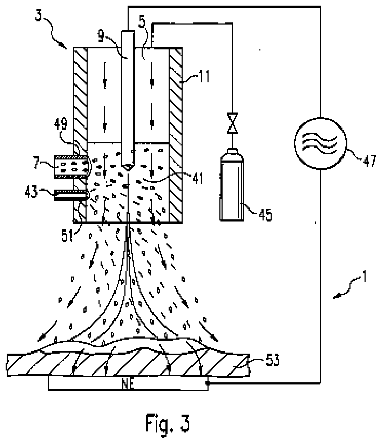

Figur 3 zeigt eine schematische Schnittdarstellung einer dritten Ausführungsform einer Karbonisierungsverhinderungsvorrichtung 1 gemäß der Erfindung. Gleiche Teile sind mit gleichen Bezugszeichen versehen, sodass insofern auf die Beschreibung zu den vorangegangenen Figuren verwiesen wird, um Wiederholungen zu vermeiden.

Die in Figur 3 gezeigte Karbonisierungsverhinderungsvorrichtung 1 weist ein chirurgisches Instrument 3 zur Durchführung einer Plasma-Koagulation auf, welches einen Gaszufuhrkanal 5 und einen Oxidationsmittelzufuhrkanal 7 umfasst, die eine Zweistoffdüse bilden. Weiterhin ist eine Elektrodenspitze 9 in dem Schlauch 11 vorgesehen.

Darüber hinaus ist ein dritter Zufuhrkanal 43 vorgesehen, durch den optional Nanopartikel zu dem Gas-Oxidationsmittel-Gemisch geleitet werden. Die Nanopartikel können sich auf einer Gewebeoberfläche 53 anlagern und so eine Unterstützung der gewünschten therapeutischen Wirkung herbeiführen.

Der Gaszufuhrkanal 5 wird wie in den beiden Ausführungsformen nach Figur 1 und 2 im Wesentlichen durch den Schlauch 11 gebildet, der an eine entsprechende Gasquelle 45 angeschlossen ist. In dem Schlauch 11 ist die Elektrodenspitze 9 angeordnet, die mit einer HF-Quelle 47 verbunden ist, und die von dem Gas umströmt wird.

Der Oxidationsmittelzufuhrkanal 7 ist über eine seitliche Öffnung 49 an dem Schlauch 11 angeschlossen, sodass Oxidationsmittel über die Öffnung 49 in den Austrittsbereich 41 gelangen kann, wo es auf das Gas aus dem Gaszufuhrkanal 5 trifft, so dass das Gas-Oxidationsmittel-Gemisch in dem Austrittsbereich 41 bereitgestellt wird. Gleiches gilt im Übrigen auch für den dritten Zufuhrkanal 43. Auch Nanopartikel gelangen somit durch eine entsprechende Öffnung 51 in den Austrittsbereich 41, wo sie zusammen mit dem Oxidationsmittel und dem Gas ein Gemisch bilden.

Vorzugsweise ist die Öffnung 49 so ausgebildet, dass flüssiges Oxidationsmittel beim Austritt aus dem Oxidationsmittelzufuhrkanal 7 zerstäubt wird, sodass in dem Austrittsbereich 41 ein Aerosol, bestehend aus Oxidationsmitteltröpfchen und dem Gas, vorliegt. Denkbar ist es jedoch auch, das Oxidationsmittel bereits vor der Einleitung in den Oxidationsmittelzufuhrkanal 7 durch einen Verdampfer zu verdampfen und dem Austrittsbereich 41 einen Oxidationsmitteldampf zuzuführen. Denkbar ist es jedoch auch, das Oxidationsmittel erst in dem Austrittsbereich 41 zu verdampfen.

Weiterhin kann eine Zerstäubung von flüssigem Oxidationsmittel erst in dem Austrittsbereich 41 beispielsweise durch eine Prallplatte oder durch Ultraschall erzeugt werden.

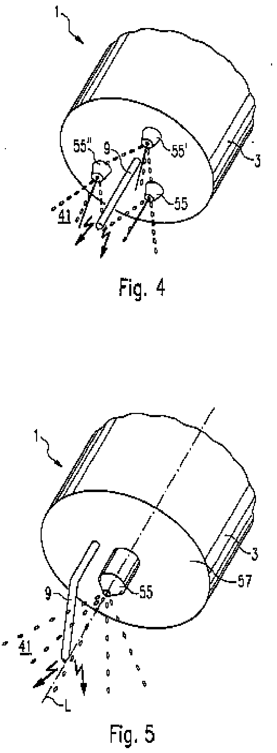

Figur 4 zeigt eine perspektivische Darstellung einer vierten Ausführungsform einer Karbonisierungsverhinderungsvorrichtung 1 gemäß der Erfindung. Gleiche Teile sind mit gleichen Bezugszeichen versehen, sodass insofern auf die Beschreibung zu den vorangegangenen Figuren verwiesen wird, um Wiederholungen zu vermeiden.

In Figur 4 ist eine zentrisch angeordnete stabförmige Elektrodenspitze 9 vorgesehen, die aus dem chirurgischen Instrument 3 herausragt. Um die Elektrodenspitze 9 herum sind drei nicht näher gezeigte Zweistoffdüsen vorgesehen, die drei Austrittsöffnungen 55, 55' und 55" aufweisen. Beispielsweise kann das chirurgische Instrument 3 wie in Figur 1 oder Figur 2 ausgebildet sein, wobei statt einer Zweistoffdüse insgesamt drei innenmischende oder außenmischende Zweistoffdüsen vorgesehen sind. Aus den Austrittsöffnungen 55, 55' und 55" strömt dann das Gas-Oxidationsmittel-Gemisch oder das Oxidationsmittel. Die Austrittsöffnungen 55, 55' und 55" sind vorzugsweise so ausgebildet, dass das Gas-Oxidationsmittel-Gemisch zerstäubt wird, sodass es in Form eines Aerosols in dem Austrittsbereich 41 vorliegt.

Figur 5 zeigt eine perspektivische Darstellung einer fünften Ausführungsform einer Karbonisierungsverhinderungsvorrichtung 1. Gleiche Teile sind mit gleichen Bezugszeichen versehen, sodass insofern auf die Beschreibung zu den vorangegangenen Figuren verwiesen wird, um Wiederholungen zu vermeiden.
In Figur 5 ist die Elektrodenspitze 9 exzentrisch bezüglich des chirurgischen Instruments 3 angeordnet und ragt in den Austrittsbereich 41 hinein. Eine Austrittsöffnung 55 einer hier nicht näher dargestellten Zweistoffdüse ist hingegen zentrisch bezüglich des chirurgischen Instruments 3 angeordnet. Die Zweistoffdüse kann auch bei dieser Ausführungsform innen- oder außenmischend ausgebildet sein.

Figur 5 macht noch deutlich, dass die Elektrodenspitze 9 derart geformt ist, dass sie von ihrer exzentrischen Austrittsposition aus in den Bereich einer Längsachse L der Austrittsöffnung 55 ragt. Der Grundkörper 57 des chirurgischen Instruments 3 ist im Übrigen vorzugsweise elektrisch isolierend ausgebildet.

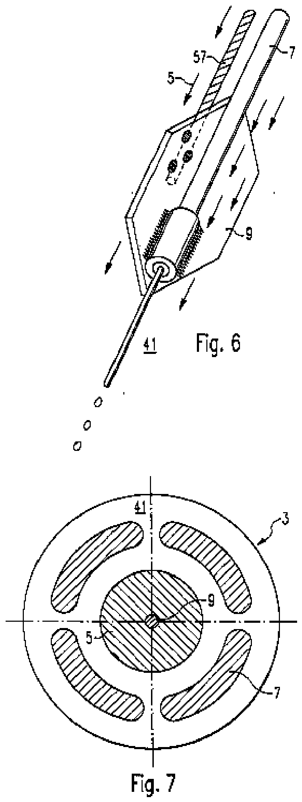

Figur 6 zeigt eine schematische Darstellung eines chirurgischen Instruments 3 mit einer Elektrode und einer Zweistoffdüse. Gleiche Teile sind mit gleichen Bezugszeichen versehen, sodass insofern auf die Beschreibung zu den vorangegangenen Figuren verwiesen wird, um Wiederholungen zu vermeiden.
In Figur 6 ist eine Zweistoffdüse vorgesehen, die außenmischend ausgebildet ist. Die Elektrodenspitze 9 ist weiterhin als Metallplatte mit einer elektrischen Zuleitung 57 ausgebildet, wobei das Gas an der Metallplatte vorbeiströmt.

Der Oxidationsmittelzufuhrkanal 7 ist an der Elektrodenspitze 9, also an der Metallplatte befestigt und erzeugt einen laminaren Strahl, sofern das Oxidationsmittel eine Flüssigkeit ist. Im Austrittsbereich 41 wird dann das Gas-Oxidationsmittel-Gemisch zur Erzeugung eines Gas-Oxidationsmittel-Plasmas bereitgestellt. Figur 7 zeigt eine Draufsicht auf einen Austrittsbereich 41 eines chirurgischen Instruments 3. Gleiche Teile sind mit gleichen Bezugszeichen versehen, sodass insofern auf die Beschreibung zu den vorangegangenen Figuren verwiesen wird, um Wiederholungen zu vermeiden.

In Figur 7 ist ein außenmischendes Zweistoffdüsensystem dargestellt, wobei die Elektrodenspitze 9 zentral angeordnet ist und von dem Gaszuführungskanal 5 umgeben ist. Koaxial zu dem Gaszuführungskanal 5 sind vier nierenförmige Oxidationsmittelzufuhrkanäle 7 vorgesehen.

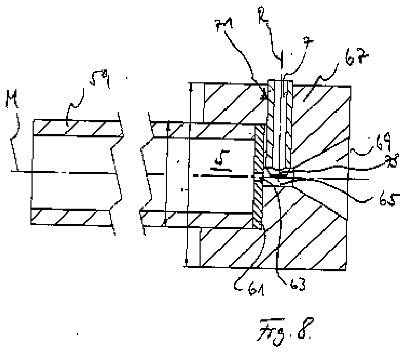

Figur 8 zeigt eine weitere Ausführungsform der Erfindung, bei der ein Gas-Oxidationsmittel-Gemisch bzw. ein Aerosol- oder Gas-Oxidationsmittel-Plasma durch das Prinzip einer (Gas-) Strahlpumpe, d.h. durch das Venturiprinzip erzeugt wird, wobei ein Unterdruck durch eine Verengung eines Zufuhrkanals erzeugt wird. Derartige Strahlpumpen sind grundsätzlich bekannt. Das Grundprinzip derartiger Pumpen besteht darin, dass aus einer Düse ein flüssiger oder gasförmiger Strahl mit hoher Geschwindigkeit austritt, der Flüssigkeit, Gas oder Feststoff aus seiner Umgebung mitreißt und beschleunigt.

Entsprechend kann gemäß der vorliegenden Erfindung ein eine Wandung 59 aufweisender Gaszufuhrkanal 5 für ein Gas, insbesondere für Argon vorgesehen sein. Das Gas strömt vorzugsweise durch eine am distalen Ende des Gaszufuhrkanals 5 angeordneten Blende 61 und insbesondere durch eine zentrische Austrittsöffnung 63 in der Blende 61 in einen zylinderförmigen Mischbereich 65 eines Aufsatzes 67, wobei der Aufsatz 67 am distalen Ende des Gaszufuhrkanals 5 angeordnet ist. Der Aufsatz 67 kann einstückig mit dem Gaszufuhrkanal 5 bzw. mit dessen Wandung 59 ausgebildet sein. Denkbar ist es aber auch den Aufsatz 67 als separates Teil auszubilden und mit dem Gaszufuhrkanal 5 auf geeignete Weise, insbesondere durch Kleben, Löten oder dergleichen, mit dem Gaszufuhrkanal 5 zu verbinden. An den zylinderförmigen Mischbereich 65 schließt sich ein kegelstumpfförmiger Zerstäuberbereich 69 an, der ebenfalls in dem Aufsatz 67 zentrisch ausgebildet ist.

Die Figur 8 macht deutlich, dass sich der Gaszufuhrkanal 5, die Öffnung 63, der Mischbereich 65 sowie der Zerstäuberbereich 69 entlang einer Mittelachse M erstrecken und im Wesentlichen symmetrisch zu dieser hintereinander angeordnet sind. Quer zu der Mittelachse M erstreckt sich entlang einer radialen Achse R ein Oxidationsmittelzufuhrrohr 71, in dem ein Oxidationsmittelzufuhrkanal 7 vorgesehen ist. Das Oxidationsmittelzufuhrrohr 71 ist mit einer nicht gezeigten Oxidationsmittelquelle verbunden. Das Oxidationsmittelzufuhrrohr 71 ist in eine entsprechende sich radial entlang der Achse R erstreckende Bohrung in dem Aufsatz 67 eingesetzt oder integral mit dieser ausgebildet und mündet mit seinem offenen Endabschnitt 73 in den distalen zylinderförmigen Mischbereich 65.

Das Funktionsprinzip der Ausführungsform nach Figur 8 ist nun wie folgt: an der Engstelle im zylindrischen Mischbereich 65 hinter der Öffnung 63 muss der statische Druck des Gases aus Energieerhaltungsgründen geringer sein als an den nicht verengten Stellen der Vorrichtung. Durch den Gasstrom in dem Gaszufuhrkanal 5 bzw. in dem Mischbereich 65 wird daher ein zu zerstäubendes Oxidationsmittel, insbesondere Wasser, aus dem Oxidationsmittelzufuhrkanal 7 durch den Unterdruck im Mischbereich 65 angesaugt und von dem Gasstrom mitgerissen. Es handelt sich somit um eine (außenmischende) selbstansaugende Zweistoffdüse bzw. um eine Venturidüse, die den Vorteil hat, dass eine separate Pumpe für die Zuführung des Oxidationsmittels entfallen kann. Vielmehr wird das Oxidationsmittel durch den Gasfluss selbsttätig in den Mischbereich 65 gesaugt. Der Gaszufuhrkanal 5 und der Oxidationsmittelzufuhrkanal 7 sind bei dieser Ausführungsform der Erfindung folglich in vorteilhafter Weise als selbstansaugende Zweistoffzerstäubereinrichtung oder als (außenmischende) Venturi-Düse ausgebildet. Ausgehend von dem Mischbereich 65 kann dann in dem Zerstäuberbereich 69 das gewünschte Gas-Oxidationsmittel-Gemisch, insbesondere als Aerosol vorliegen und durch eine geeignete Elektrode ein Gas-Oxidationsmittel-Plasma gezündet werden.

Insgesamt zeigt sich, dass die vorliegende Erfindung eine Karbonisierungsverhinderungsvorrichtung 1 schafft, die in vorteilhafter Weise ein Gas-Oxidationsmittel-Gemisch zur Erzeugung eines Gas-Oxidationsmittel-Plasmas mittels eines chirurgischen Instruments 3 bereitstellt.

Das Gas-Oxidationsmittel-Gemisch weist mindestens zwei Komponenten auf, wobei die eine Komponente ein Gas, insbesondere ein Edelgas wie beispielsweise Argon oder Helium und die andere Komponente ein Oxidationsmittel für Kohlenstoff ist. Das Oxidationsmittel kann dabei aus festen oder flüssigen Schwebeteilchen, z.B. kleinen Wassertröpfchen bestehen, die als Wassernebel vorliegen. Hierbei wird flüssiges Oxidationsmittel sehr fein zerstäubt, so dass sich seine Oberfläche stark vergrößert. Auf diese Weise wird der Verdampfungspunkt erheblich herabgesetzt, so dass neben den flüssigen Oxidationsmitteltröpfchen ein erheblicher Anteil an Oxidationsmitteldampf vorliegt. Durch den hochfrequenten Wechselstrom können auch Oxidationsmittelmoleküle, insbesondere Wassermoleküle in der Gasphase zu einem Wasserdampfplasma-Gemisch ionisiert werden.

Die obigen Ausführungen machen deutlich, dass das Gas-Oxidationsmittel-Gemisch vorzugsweise ein Aerosol ist, welches also gasförmige Teilchen und fein zerstäubte Oxidationsmitteltröpfchen aufweist. Mit dem Aerosolplasma soll weitgehend eine Karbonisierung des Gewebes vermieden werden, wobei der Oxidationsmittelnebel, insbesondere der Wassernebel, d.h. die H20-Tröpfchen gleichzeitig als Oxidationsmittel für Kohlenstoff, als Kühlmittel für die Gewebeoberfläche und als Plasmamedium wirkt.

Weiterhin ist die erhebliche Verminderung der Karbonisierung direkt mit der Emissionsmenge von Ruß und Rauchgasen wie C02, CO, NO, NOx, SOx, organischen und biochemischen Molekülen verbunden, so dass die vorgeschlagene Vorrichtung und das entsprechende Verfahren eine deutliche Verringerung obengenannter Emissionen zur Folge hat und damit die Expositionsrisiken von Patient und OP-Personal senkt.

Außerdem wird mit der vorgeschlagenen Karbonisierungsverhinderungsvorrichtung eine homogene und gewebeschonende Koagulation und Devitalisierung erzielt, welche darauf abzielt, das Verfahren schonend vorzugsweise im Bereich der Onkochirurgie, aber auch anderen medizinischen Disziplinen, z.B. zur Tumorablation, insbesondere bei dünnwandigen und nervsensiblen Strukturen, in der Neurochirurgie, Urologie und als adhäsionsreduzierende Chirurgiemethode in der Gynäkologie und Visceralchirurgie sowohl offenchirurgisch als auch endoskopisch (starr und flexibel) einzusetzen.

Weiterhin kann wenigstens eine Zweistoffdüse vorgesehen sein, die innenmischend oder außenmischend ausgebildet sein kann. Darüber hinaus kann das chirurgische Instrument 3 zur Erzeugung eines Oxidationsmittel-Aerosols bzw. eines Gas-Oxidationsmittel-Aerosols über geeignete Mittel verfügen, beispielsweise über einen Verdampfer, einen Ultraschallerzeuger oder über eine Prallplatte. Das Oxidationsmittel kann dabei entweder vor oder nach der Zusammenführung mit dem Gas zerstäubt werden. Entscheidend ist lediglich, dass flüssige Oxidationsmitteltröpfchen in dem Gas vorhanden sind, um die oben beschriebenen Vorteile zu bewirken. Die vorliegende Erfindung vermindert damit effektiv eine Karbonisierung und eine Ruß- und Rauchentwicklung. Weiterhin wird eine gleichmäßigere Verteilung der Plasmaenergie auf der Gewebeoberfläche erzielt. Die oben genannten Vorteile werden auch durch ein erfindungsgemäßes Verfahren erzielt, welches ein Gas-Oxidationsmittel-Gemisch für die Durchführung einer Plasma-Koagulation bereitstellt. Gleiches gilt im Übrigen für die Verwendung einer Karbonisierungsverhinderungsvorrichtung 1 zur Verhinderung einer Karbonisierung des Gewebes.

### Bezugszeichenliste

I Karbonisierungsverhinderungsvorrichtung
3 Chirurgisches Instrument
5 Gaszufuhrkanal
7 Oxidationsmittelzufuhrkanal
9 Elektrodenspitze
II Schlauch
13 Isolationsschutz
15 Fixierhülse
17 Innenwandung
19 Rastvorsprung
21 Distaler Bereich
3 Distales Ende (Schlauch)
5 Öffnung (Schlauch)
7 Rohr
9 Distales Ende (Rohr)
1 Distales Ende (Elektrodenspitze)
3 Ringraum
5 Öffnung (Zufuhrkanal)
7 Diffusor
9 Durchgangsbohrung
1 Austrittsbereich
3 Dritter Zufuhrkanal
5 Gasquelle
7 HF-Quelle
9 Öffnung (Oxidationsmittel) 51 Öffnung (Nanopartikel)
53 Gewebeoberfläche
55 Austrittsöffnung
55' Austrittsöffnung
55" Austrittsöffnung
57 Elektrische Zuleitung
59 Wandung
61 Blende
63 Austrittsöffnung
65 Mischbereich
67 Aufsatz
69 Zerstäuberbereich
71 Oxidationsmittelzufuhrrohr
73 Endabschnitt
D Innendurchmesser
L Längsachse
M Mittelachse
R Radiale Achse

## Patentansprüche

1. Instrument (3) mit einer Karbonisierungsverhinderungsvorrichtung (1) zum Verhindern der Karbonisierung von Gewebe bei einer Argon-Plasma-Koagulation mittels eines geeigneten chirurgischen Instruments (3), wobei das chirurgische Instrument (3) einen in einem Rohr (27) ausgebildeten Oxidationsmittelzufuhrkanal (7) zur Zuführung für Wasser als Oxidationsmittel (7), einen Gaszufuhrkanal (5), der durch einen Schlauch (11) gebildet ist, der an eine Gasquelle (45) angeschlossen ist zur Zuführung für Argon (5), und eine Elektrodenspitze (9) zur Erzeugung eines Plasmas aufweist, **dadurch gekennzeichnet, dass** das Rohr (27) an seinem proximalen Ende mit einer HF-Spannungsquelle verbunden und das distale Ende (29) des Rohrs (27) mit der Elektrodenspitze (9) verbunden ist, um die Elektrodenspitze (9) mit einem hochfrequenten Strom zu versorgen, wobei das Rohr (27) mit einer Quelle für das Oxidationsmittel Wasser verbunden ist, so dass das Wasser durch das Rohr (27) und durch die Elektrodenspitze (9) zu einem distalen Ende (31) der Elektrodenspitze (9) gelangen kann, und wobei durch die Karbonisierungsverhinderungsvorrichtung (1) mittels einer Zweistoffzerstäubereinrichtung, die durch den Gaszufuhrkanal und den Oxidationsmittelzufuhrkanal gebildet wird, ein Argon-Wasser-Aerosol zur Erzeugung eines Argon-Wasser-Plasmas bereitgestellt wird.

2. Instrument nach Anspruch 1, **dadurch gekennzeichnet, dass** das chirurgische Instrument (3) zur Erzeugung des Aerosols einen Verdampfer aufweist.

3. Instrument nach Anspruch 1, **dadurch gekennzeichnet, dass** das chirurgische Instrument (3) zur Erzeugung des Aerosols eine Ultraschallerzeugungseinrichtung aufweist.

4. Instrument nach Anspruch 1, **dadurch gekennzeichnet, dass** das chirurgische Instrument (3) zur Erzeugung des Aerosols eine Prallfläche aufweist.

5. Instrument nach einem der vorhergehenden Ansprüche, **dadurch gekennzeichnet, dass** mindestens eine Zweistoffzerstäubereinrichtung vorgesehen ist.

6. Instrument nach Anspruch 5, **dadurch gekennzeichnet, dass** die mindestens eine Zweistoffzerstäubereinrichtung innenmischend ausgebildet ist.

7. Instrument nach Anspruch 5, **dadurch gekennzeichnet, dass** die mindestens eine Zweistoffzerstäubereinrichtung außenmischend ausgebildet ist.

8. Instrument nach einem der vorhergehenden Ansprüche, **dadurch gekennzeichnet, dass** das chirurgische Instrument (3) einen Schlauch (11) aufweist, der im Bereich der Elektrode (9) wenigstens eine Öffnung (25) zur Verhinderung einer Embolie aufweist.

## Claims

1. Instrument (3) with an anti-carbonisation device (1) for preventing the carbonisation of tissue during argon-plasma coagulation by means of a suitable surgical instrument (3), wherein the surgical instrument (3) comprises a supply channel for oxidation agent (7) formed in a pipe (27) and intended for supplying water as an oxidation agent (7), a supply channel for gas (5) which is formed by a hose (11) connected to a gas source (45) for supplying argon (5), and an electrode tip (9) for producing a plasma, **characterised in that** the pipe (27) is connected at its proximal end to an HF voltage source, and the distal end (29) of the pipe (27) is connected to the electrode tip (9) in order to supply the electrode tip (9) with a high-frequency current, wherein the pipe (27) is connected to a source for water as the oxidation agent so that the water can pass through the pipe (27) and the electrode tip (9) to a distal end (31) of the electrode tip (9), and wherein an argon-water aerosol is provided by the anti-carbonisation device (1) in order to generate an argon-water plasma by means of a two-substance atomisation device which is formed by the gas supply channel and the oxidation agent supply channel.

2. Instrument according to claim 1, **characterised in that** to generate the aerosol, the surgical instrument (3) has an evaporator.

3. Instrument according to claim 1, **characterised in that** to generate the aerosol, the surgical instrument (3) has an ultrasound production device.

4. Instrument according to claim 1, **characterised in that** to generate the aerosol, the surgical instrument (3) has a rebound surface.

5. Instrument according to any of the preceding claims, **characterised in that** at least one two-substance atomisation device is provided.

6. Instrument according to claim 5, **characterised in that** the at least one two-substance atomisation device is configured with internal mixing.

7. Instrument according to claim 5, **characterised in that** the at least one two-substance atomisation device is configured with external mixing.

8. Instrument according to any of the preceding claims, **characterised in that** the surgical instrument (3) comprises a hose (11) which has at least one opening (25) in the region of the electrode (9) for preventing an embolism.

## Revendications

1. Instrument (3) doté d'un dispositif anticarbonisation (1) destiné à empêcher la carbonisation de tissus lors d'une coagulation par plasma d'argon à l'aide d'un instrument chirurgical (3) approprié, l'instrument chirurgical (3) présentant une conduite d'alimentation en agent oxydant (7), réalisée dans un tuyau rigide (27), pour amener de l'eau en tant qu'agent oxydant (7), une conduite d'alimentation en gaz (5), qui est constituée d'un tuyau flexible (11) raccordé à une source de gaz (45) en vue de l'amenée d'argon (5), et une pointe d'électrode (9) destinée à produire un plasma, **caractérisé en ce que** le tuyau rigide (27) est relié par son extrémité proximale à une source de tension HF, et que l'extrémité distale (29) du tuyau rigide (27) est reliée à la pointe d'électrode (9) afin d'alimenter la pointe d'électrode (9) avec un courant de haute fréquence, le tuyau rigide (27) étant relié à une source pour l'agent oxydant constitué d'eau, de sorte que l'eau peut parvenir à travers le tuyau rigide (27) et à travers la pointe d'électrode (9) jusqu'à une extrémité distale (31) de la pointe d'électrode (9), et le dispositif anticarbonisation (1) fournissant, au moyen d'un dispositif atomiseur à deux fluides qui est constitué de la conduite d'alimentation en gaz et de la conduite d'alimentation en agent oxydant, un aérosol d'argon-eau destiné à produire un plasma d'argon-eau.

2. Instrument selon la revendication 1, **caractérisé en ce que** l'instrument chirurgical (3) présente un vaporisateur en vue de la production de l'aérosol.

3. Instrument selon la revendication 1, **caractérisé en ce que** l'instrument chirurgical (3) présente un dispositif générateur d'ultrasons en vue de la production de l'aérosol.

4. Instrument selon la revendication 1, **caractérisé en ce que** l'instrument chirurgical (3) présente une surface déflectrice en vue de la production de l'aérosol.

5. Instrument selon une des revendications précédentes, **caractérisé en ce qu'**il est prévu au moins un dispositif atomiseur à deux fluides.

6. Instrument selon la revendication 5, **caractérisé en ce que** le dispositif atomiseur à deux fluides, au nombre d'au moins un, est réalisé de manière à effectuer le mélange de façon interne.

7. Instrument selon la revendication 5, **caractérisé en ce que** le dispositif atomiseur à deux fluides, au nombre d'au moins un, est réalisé de manière à effectuer le mélange de façon externe.

8. Instrument selon une des revendications précédentes, **caractérisé en ce que** l'instrument chirurgical (3) présente un tuyau flexible (11) qui comporte, dans la région de l'électrode (9), au moins une ouverture (25) destinée à empêcher une embolie.
